# EUROPEAN PATENT APPLICATION

(11) **EP 0 653 490 A1**
(43) Date of publication of application: **17.05.1995**
(21) Application number: 93913573.7
(22) Date of filing: 24.06.1993
(51) Int. Cl.: C12N 15/53, C12N 1/11

(54) **VITAMIN D HYDROXYLASE GENE**

(30) Priority: 25.06.1992 JP 167644/92
(71) Applicant: TAISHO PHARMACEUTICAL CO. LTD, Tokyo 171 (JP)
(72) Inventor: KAWAUCHI, Hiroyuki Taisho Pharma. Co.,Ltd., Toshima-ku Tokyo 171 (JP); SASAKI, Joji Taisho Pharmaceutical Co., Ltd., Toshima-ku Tokyo 171 (JP); ADACHI, Takashi Taisho Pharma.Co., Ltd., Toshima-ku Tokyo 171 (JP); HANADA, Kazunori Taisho Pharma.Co., Ltd., Toshima-ku Tokyo 171 (JP); BEPPU, Teruhiko, Tokyo 166 (JP); HORINOUCHI, Sueharu, Tokyo 135 (JP)
(74) Representative: Weisert, Annekäte, Dipl.-Ing. Dr.-Ing.
(86) International application number: JP9300856
(87) International publication number: WO9400576

(57) **Abstract**

A gene having the activity of a hydroxylase that can convert the hydrogen atom at the 25-position of a vitamin D into a hydroxyl group is obtained from the strain of an actinomyces of the genus *Nocardia* or *Amycolata*. A 25-hydroxylated vitamin D can be prepared efficiently in a high yield by transforming *Streptomyces lividans* or *Streptomyces griseus* with a plasmid bearing the above gene and treating a vitamin D having a hydrogen atom at the 25-position with the resultant transformant.

## Description

### Technical Field

The present invention relates to a gene responsible for a hydroxylase activity, which is present in bacteria belonging to Actinomycetes, especially to the genus Nocardia or the genus Amycolata, and isolation of a DNA bearing the gene as well as use thereof.

More particularly, the present invention relates to a gene responsible for expression of a hydroxylase activity which is present in bacteria belonging to Actinomycetes, especially to the genus Nocardia or the genus Amycolata and which encodes a protein having a hydroxylase activity which is capable of converting the hydrogen atom at the 25-position of a vitamin D compound having a hydrogen atom at the 25 position into a hydroxyl group, or a DNA bearing the gene; the gene responsible for the expression of the hydroxylase activity existing in a DNA fragment of about 3.83 kb or a DNA bearing the aforesaid gene is obtained by partial digestion of chromosomal DNA from the cells of Actinomycetes with restriction enzyme Sau 3AI.

### Background Art

It is known to hydroxylate the 1- and 25-positions of vitamin D compounds enzymologically using microorganism (microbiological conversion) (Japanese Patent Application KOKAI Nos.2-469 and 2-231089). However, a gene of microorganism origin which is responsible for the hydroxylase activity of a vitamin D compound is unknown heretofore. It was thus insufficient as a technology to increase the amount of a hydroxylase responsible for the hydroxylase activity of a vitamin D compound in bacteria or to produce the hydroxylase in an industrial scale. In animal, a gene for vitamin D₃ 25-hydroxylase cytochrome P-450 from rat liver is known, including its cloning (Japanese Patent Application KOKAI No. 3-232493). However, the enzyme activity of the cloned gene was insufficiently expressed in microorganisms. A recent report reveals that the same gene from rat liver expressed in cytoplasmic reticulum of yeast increased the expression of the activity in microorganism approximately 50 times larger than in the method previously disclosed ("JISEDAI SANGYO KIBAN SYMPOSIUM" (Symposium of Principal Industry in the Next Generation), held September 13, 1991). However, the yield does not yet reach the level obtained by conversion using a microorganism having a vitamin D hydroxylase activity.

Even though the gene for vitamin D 25-hydroxylase cytochrome P-450 from rat liver is cloned and successfully expressed in a microorganism, the yield is not comparable to that obtained by conversion using a microorganism having a vitamin D hydroxylase activity of microorganism origin. This is considered to be because the enzyme activity per molecule of the hydroxylase derived from a microorganism is much better than the hydroxylase of animal origin. It has thus been desired to provide a recombinant DNA bearing a gene capable of expressing a vitamin D hydroxylase derived from a microorganism and to acquire a transformant bearing a gene capable of expressing a vitamin D hydroxylase derived from a microorganism to conserve a recombinant DNA capable of self-amplification in the microorganism.

### Disclosure of Invention

The present inventors have made studies to provide a gene responsible for the production of a specific enzyme of Actinomycetes belonging to the genus Nocardia or the genus Amycolata, which can be used to catalyze the microbial conversion of a vitamin D compound, or a DNA bearing the gene. As a result, a gene being capable of expressing a hydroxylase activity which can convert the 25 hydrogen of a vitamin D compound containing a hydrogen atom at the 25 position thereof into a hydroxyl group to produce a 25-hydroxyvitamin D compound could be successfully isolated from microorganisms belonging to the genus Nocardia or the genus Amycolata. Furthermore, it has been found that by inserting a plasmid incorporated with the gene into Streptomyces lividans or Streptomyces griseus, deleted the hydroxylation activity which is capable of converting a vitamin D compound having a hydrogen atom at the 25-position (at 25-position thereof) into a 25-hydroxyvitamin D compound or which has the low hydroxylation activity, the gene can be expressed and can convert vitamin D compounds having a hydrogen atom at the 25 position into 25-hydroxyvitamin D compounds more efficiently than its original strain of the gene.

According to the present invention, there are provided a gene encoding a hydroxylase derived from Actinomycetes which can convert the 25 hydrogen of a vitamin D compound containing hydrogen atom at the 25 position thereof into a hydroxyl group, a recombinant plasmid bearing a DNA fragment of the gene, and a transformant bearing the plasmid.

### Brief Description of Drawings

Fig. 1 is a restriction map of a DNA fragment of about 3.83 kbp bearing a gene for expressing a hydroxylase activity which is capable of converting a vitamin D compound into a 25-hydroxy-vitamin D compound by catalyzing the hydroxylation of the 25 position of a vitamin D compound. In the figure, Hin dIII, Sac I, Bcl I, Kpn I, Pst I and Bam HI represent restriction enzymes, respectively and numerical figures indicate a distance between the respective sites for restriction enzymes in terms of kbp.

Fig. 2 shows recombinant plasmid PHK010 and a plasmid from which the recombinant plasmid originated. Digestion of plasmid PIJ699(1) with restriction enzyme Bgl II followed by treatment of DNA fragment (2) by T4 DNA ligase results in ligation of the DNA fragment (2) to give plasmid PHK010 (3).

Fig. 3 shows a restriction map of recombinant plasmid PHK030. Numerals within parentheses indicate sites for the respective restriction enzymes, where the digestion site of restriction enzyme Hin dIII located at the site having restriction enzyme Sac I site therearound is made the coordinate point. The fine line indicates a portion homologous to the DNA fragment derived from plasmid PHK010 using as a vector plasmid. The bold line indicates a portion homologous to the DNA fragment bearing the gene having a hydroxylase activity shown in Fig. 1.

Fig. 4 shows a restriction map of recombinant plasmid PHK031. The numeral figures within parentheses indicate sites for the respective restriction enzymes, where the digestion site of restriction enzyme Sau 3AI located at the site having restriction enzyme Sac I site therearound is made the coordinate point. The fine line indicates a portion homologous to the DNA fragment derived from plasmid PIJ702 using as a vector plasmid. The bold line indicates a portion homologous to the DNA fragment bearing the gene having a hydroxylase activity shown in Fig. 1.

Fig. 5 shows the former half of the nucleotide sequence of 3836 bp DNA fragment bearing the gene encoding hydroxylase derived from chromosomal DNA of Nocardia autotrophica N-102 strain cloned on plasmid PHK030.

Fig. 6 shows the latter half of the nucleotide sequence of 3836 bp DNA fragment bearing the gene encoding hydroxylase derived from chromosomal DNA of Nocardia autotrophica N-102 strain cloned on plasmid PHK030.

Fig. 7 shows a site for expressing vitamin D₃ 25 hydroxylation activity in the 3836 bp DNA fragment bearing the gene encoding hydroxylase cloned on plasmid PHK030.

Fig. 8 shows the former half of the nucleotide sequence of the gene encoding hydroxylase.

Fig. 9 shows the latter half of the nucleotide sequence of the gene encoding hydroxylase.

### Best Mode for Carrying Out the Invention

The gene of the present invention encoding a hydroxylase or the DNA fragment bearing the gene may be any gene for also catalyzing the hydroxylation of other substrates, so long as the gene can convert the hydrogen atom at the 25-position of vitamin D compounds having a hydrogen atom at the 25 position into a hydroxyl group to produce 25-hydroxyvitamin D compounds. As sources of the gene encoding a hydroxylase or the DNA fragment containing the gene, those derived from chromosomal DNA of Actinomycetes capable of converting the hydrogen atom at the 25-position of a vitamin D compound containing hydrogen atom at the 25 position into a hydroxyl group to produce a 25-hydroxyvitamin D compound may be preferably employed. Examples of such sources include Nocardia autotrophica (currently classified generally into Amycolata autotrophica) and, Amycolata saturnea, etc.

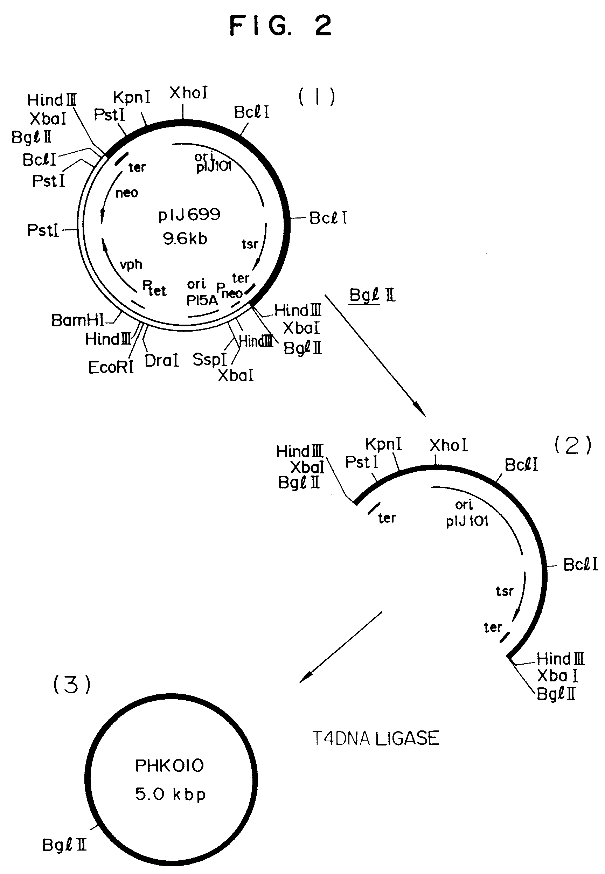

On the other hand, any vector plasmid may be used for introducing the gene encoding hydroxylase or the DNA fragment bearing the gene as far as it belongs to Actinomycetes, capable of self-amplification and has an excellent stability to conserve and stably transfer its ability to daughter cells in division of host cells. The vector plasmid can thus be freely chosen depending upon host used. Specific examples of the vector plasmid include PHK010 shown in Fig. 2, obtained by digesting known PIJ702 (Katz et al., J. Gen. Microbiol., 129, 2703 (1983)) or PIJ699 (Kieser et al., Gene, 65, 83 (1988)) with restriction enzyme Bgl II, etc.

The gene encoding a hydroxylase or the DNA fragment bearing the gene which is provided by the present invention is inserted into the vector plasmid described above to obtain plasmid PHK030 or PHK031 named by the inventor and later described.

The gene encoding a hydroxylase which is provided by the present invention is used to mean DNA responsible for the production of a hydroxylase which catalyzes the reaction of producing 25-hydroxyvitamin D compounds by converting the hydrogen atom at the 25-position of vitamin D compounds having a hydrogen atom at the 25-position into hydroxyl group.

Examples of the vitamin D compound having a hydrogen atom at the 25 position which is used in the present invention are vitamin D₂ compounds, vitamin D₃ compounds, vitamin D₄ compounds, vitamin D₅ compounds, vitamin D₆ compounds, vitamin D₇ compounds, and the like. These compounds may also be those substituted the side chain at the 17-position with a hydrogen atom, a hydroxyl group, a lower alkyl group, etc. Specific examples include vitamin D₂, vitamin D₃, vitamin D₄, vitamin D₅, vitamin D₆, vitamin D₇, 24-oxovitamin D₃, 1α-hydroxyvitamin D₂, 1α-hydroxyvitamin D₃, 1α-hydroxyvitamin D₄, 1α-hydroxyvitamin D₅, 1α-hydroxyvitamin D₆, 1α-hydroxyvitamin D₇, 1α-hydroxy-24-oxovitamin D₃, 24-hydroxyvitamin D₃, 23-hydroxyvitamin D₃, 23-hydroxyvitamin D₄, 1α,23-dihydroxyvitamin D₃, 1α,23-dihydroxyvitamin D₄, 26-hydroxyvitamin D₂, 26-hydroxyvitamin D₄, 23,24-dihydroxyvitamin D₃, 23,26-dihydroxyvitamin D₃, 23,26-dihydroxyvitamin D₄, vitamin D₃, 26,23-lactone, 1α-hydroxyvitamin D₃, 26,23-lactone, 24,24-difluorovitamin D₃, 24,24-dichlorovitamin D₃, 26,26,26,27,27,27-hexafluorovitamin D₃, 24,24-difluoro-25-hydroxy-26,27-dimethylvitamin D₃, etc.

The recombinant plasmid bearing the gene encoding the hydroxylase or the DNA containing the gene according to the present invention may be prepared in a conventional manner (e.g., D.A. Hopwood et al., "Genetic Manipulation of Streptomyces", A Laboratory Manual, The John Innes Foundation, 1985).

### Preparation of recombinant plasmid

### 1) Vector plasmid

Any vector plasmid may be employed so long as plasmids belong to Actinomycetes and can stably maintain replication and amplification. The vector plasmids may also include those derived from these vector plasmids depending upon purpose of use. As the vector, there may be used, for example, PHK010 (Fig. 2) obtained by digesting known plasmid, e.g., PIJ702 or PIJ699 with restriction enzyme Bgl II using Streptomyces lividans TA-220 (FERM P-12962, Fermentation Research Institute of the Agency of Industrial Science & Technology Japan) or Streptomyces griseus (FERM P-12963, Fermentation Research Institute of the Agency of Industrial Science & Technology Japan). To these plasmids are imparted thiostrepton resistance (hereinafter referred to as "Thior^{r}") as a marker. By selecting transformants showing Thior^{r}, recombinant plasmid can be readily produced.

### 2) Cloning of DNA bearing the gene for expressing vitamin D hydroxylase activity

Chromosomal DNA is extracted from cells of Actinomycetes (e.g., Nocardia autotrophica, etc.) containing the gene for expressing a hydroxylase activity, which can hydroxylate the 25-position of the aforesaid vitamin D compounds having hydrogen at the 25-position to convert into 25-hydroxyvitamin D compounds, by known methods, e.g., the method of Murmur (J. Mol. Biol., 3, 208 (1961)). The extracted DNA is digested with a suitable restriction enzyme (commercially available, e.g., from Takara Shuzo Co. or Wako Pure Chemical Industry) to obtain the DNA fragment bearing the desired gene together with other DNA fragments. From the thus obtained mixture of DNA fragments, the recombinant plasmid containing DNA bearing the desired gene can be prepared as follows. Firstly, the DNA fragment containing the desired gene is incorporated into a vector plasmid which is previously treated so that the plasmid can be ligated with the terminus of the DNA fragment. Host is then transformed with the thus formed recombinant plasmids. Then, transformants containing recombinant plasmid showing Thior^{r} are selected. From the so selected transformants, a transformant for expressing the desired character is selected, thereby to select a recombinant plasmid containing DNA bearing the desired gene.

For example, chromosomal DNA of Nocardia autotrophica is partially digested into DNA fragment of 3-20 kb with restriction enzyme Sau 3AI (e.g., T. Maniatis et al., "Molecular Cloning", Cold Spring Harbor Laboratory, page 282, 1982). The resulting DNA fragment is blended with PHK010 cleaved and linear-fashioned with restriction enzyme Bgl II followed by ligation of the DNA fragment and PHK010 with T4 DNA ligase (commercially available; Takara Shuzo Co., etc.). By this procedure there is obtained the ligation product containing the desired recombinant plasmid in which the fragment of the chromosomal DNA has been inserted. To select the desired recombinant plasmid from the ligation product, the product is inserted into protoplast of Actinomycetes deleted of a hydroxylase activity of converting the vitamin D compounds into 25-hydroxy-vitamin D compounds or having the low hydroxylase activity, which is smeared on an agar plate. That is, a mixture of the protoplast and soft agar medium added with thiostrepton is overlaid on the agar plate. After overlaying, the agar plate is incubated. The transformant containing the recombinant plasmid grows into colonies due to resistance to thiostrepton. From the colonies, the transformant capable of expressing the desired character is selected.

That is, the mycelia of each colony are inoculated on liquid medium and sufficiently grown by aerated spinner culture. Next, vitamin D₃ is added to the culture broth in which the mycelia grown. Fermentation with aeration and agitation follows solvent extraction by a modification of the Bligh and Dyer method. The extract may be analyzed in a manner similar to known vitamin D analysis (e.g., Methods in Enzymology, 123, 127-154 (1986)). By comparing retention time in high performance liquid chromatography using regular phase silica gel column (e.g., Zorbax-SIL, Du Pont, USA), UV absorption spectrum and EI-MS spectrum of the product with those of authentic product (25-hydroxyvitamin D₃ (25(OH)D₃), Duphar Co., Holland), it can be confirmed if 25(OH)D₃ is present in the extract.

By the above procedures, there can be selected a colony in which the formation of 25(OH)D₃ has been confirmed, namely, a colony containing the recombinant plasmid bearing DNA with the gene encoding a hydroxylase having an activity of catalyzing hydroxylation of vitamin D compounds having hydrogen at the 25-position to convert into 25-hydroxyvitamin D compounds. Accordingly, the recombinant plasmid containing DNA bearing the gene encoding a hydroxylase having an activity of catalyzing hydroxylation of vitamin D compounds having hydrogen at the 25 position to convert into 25-hydroxyvitamin D compounds can be obtained from the cells of this colony by the plasmid extraction procedures described above. Examples of the thus obtained recombinant plasmid include PHK030 and PHK031.

### 3) Specific description on recombinant plasmid

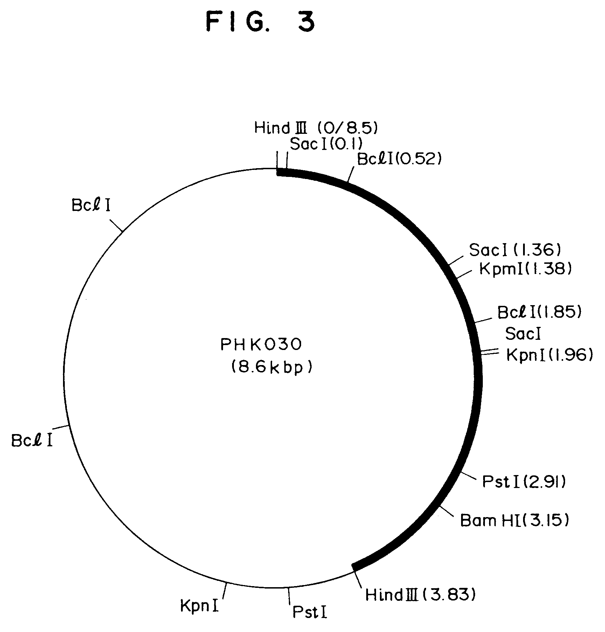

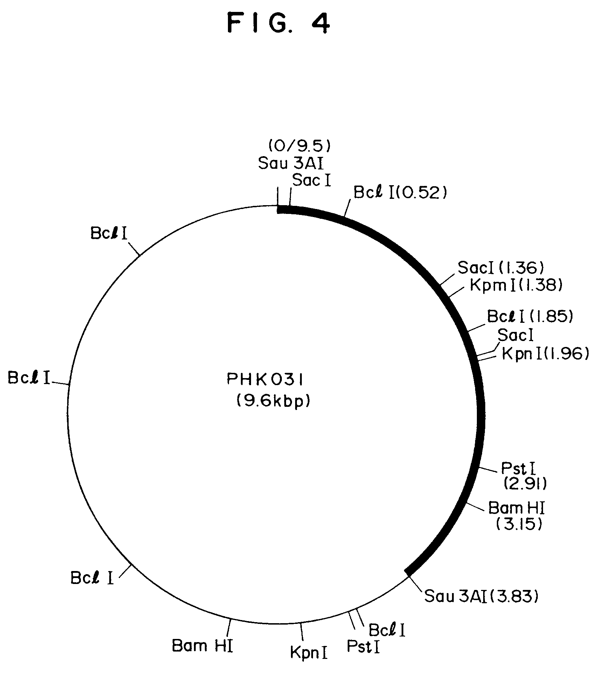

Recombinant plasmids pHK030 and PHK031 (cf. Working Examples and Figs. 3 and 4) obtained by the method described above are specifically described hereinbelow.

### (1) Confirmation of hydroxylase activity by plasmids PHK030 or PHK031

It is understood from the following that the inserted DNA fragment containing plasmid PHK030 or PHK031 contains the gene encoding a hydroxylase having the activity of catalyzing hydroxylation of vitamin D compounds into 25-hydroxyvitamin D compounds. That is, it is understood from the fact that the plasmid-free strain obtained by removing the plasmid from the recombinant bearing plasmid PHK030 or PHK031 (for example, treatment of the recombinant with acridine dye, etc.) becomes sensitive to thiostrepton and at the same time, looses the hydroxylation activity of vitamin D compounds. In addition, re-transformants obtained by retransformation by plasmid PHK030 or PHK031 using the plasmid-free strain as a host all express Thior^{r} and acquires again the hydroxylation activity of vitamin D compounds.

### (2) Plasmid PHK030

Plasmid PHK030 having a restriction map shown in Fig. 3 can be digested by restriction enzyme Hin dIII into DNA fragments of 3.8 kbp and 4.7 kbp. The site for digestion with restriction enzyme Sau 3AI is located within several tens bp from the termini of the 3.8 kbp DNA fragment, and the gene encoding the hydroxylase derived from the chromosome of Nocardia autotrophica is contained inward the site. Another DNA fragment is a region derived from plasmid PHK010 (5.0 kbp, Fig. 2). PHK010 is contained in PHK030 as the DNA fragment which is lost by 200 to 300 bp through recombination. Plasmid PHK030 may be prepared from a transformant bearing the plasmid alone.

### (3) Plasmid PHK031

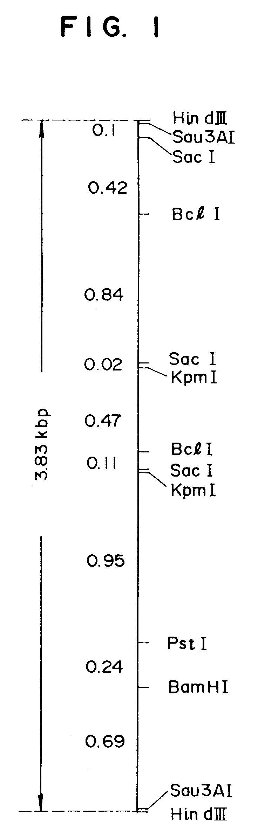

Fig. 4 shows a restriction map for plasmid PHK031. Plasmid PHK031 is a plasmid having a size of 9.5 kbp obtained by ligating the DNA fragment resulting from digestion and ring-opening of plasmid PIJ702 with restriction enzyme Bgl II and the DNA fragment of about 3.8 kbp, obtained by digestion of plasmid PHK030 with restriction enzyme Sau 3AI, using T4 DNA ligase. Plasmid PHK031 may be prepared from a transformant containing the plasmid alone. Furthermore by acting restriction enzyme Sau 3AI on plasmid PHK031 and binding the resulting DNA fragment of about 3.8 kbp to plasmid PHK010 linear-fashioned with restriction enzyme Bgl II, the plasmid which is perfectly identical with plasmid PHK030 having the same restriction map for plasmid PHK030, can be obtained. The hydroxylation activity of vitamin D compounds has been confirmed both in plasmids PHK030 and PHK031. Accordingly, it has been confirmed that the gene encoding hydroxylase is contained in the DNA fragment of about 3.83 kbp obtained by acting restriction enzyme Hin dIII on plasmid PHK030. This DNA fragment of about 3.83 kbp has a restriction map shown in Fig. 1.

### Determination of nucleotide sequence of the gene encoding hydroxylase

The DNA fragment of about 3.83 kbp bearing the gene encoding a hydroxylase cloned on, e.g., plasmid PHK030 described above is obtained. Using this DNA fragment, a nucleotide sequence of the gene encoding a hydroxylase can be determined by the dideoxy chain terminator method using, e.g., SEQUENASE DNA Sequencing Kit.

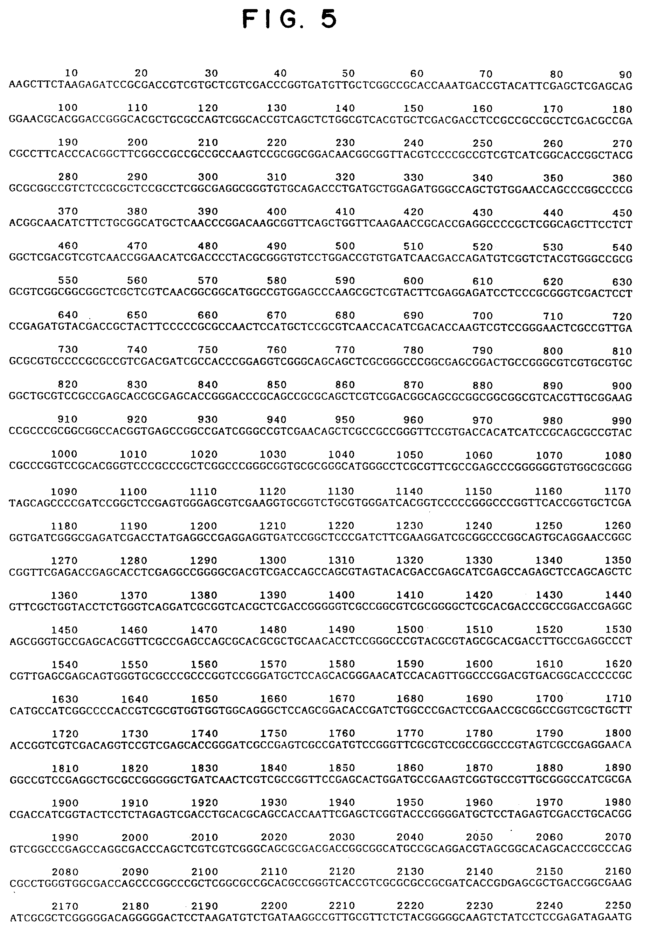

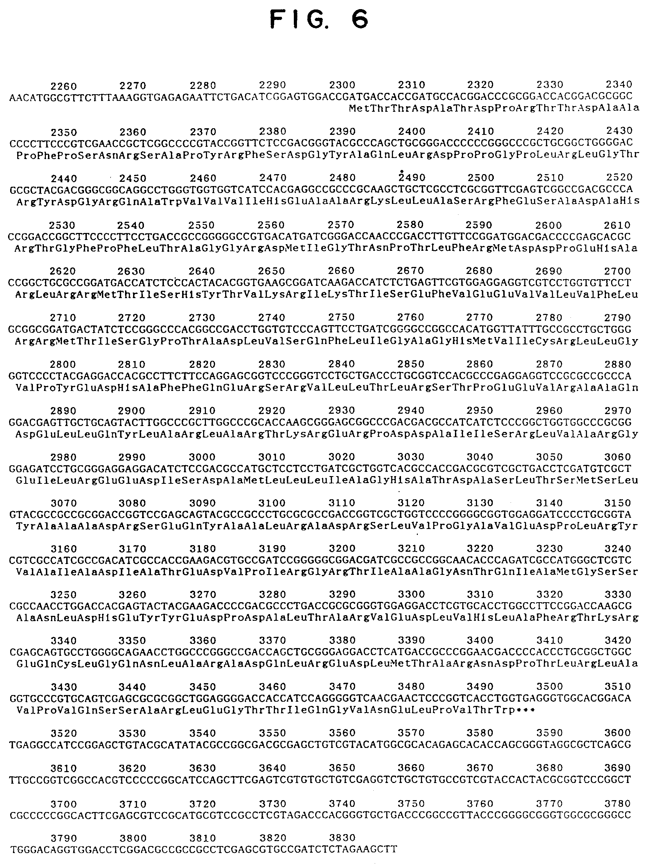

As will be shown in Example 8 later described, the DNA fragment derived from chromosomal DNA of Nocardia autotrophica cloned on plasmid PHK030 has a size of 3836 bp. It is revealed that the DNA fragment has initiation codon at 2300 bp from the upstream and has an open reading frame of 1194 bp comprising 398 amino acids (cf. Figs. 5 and 6).

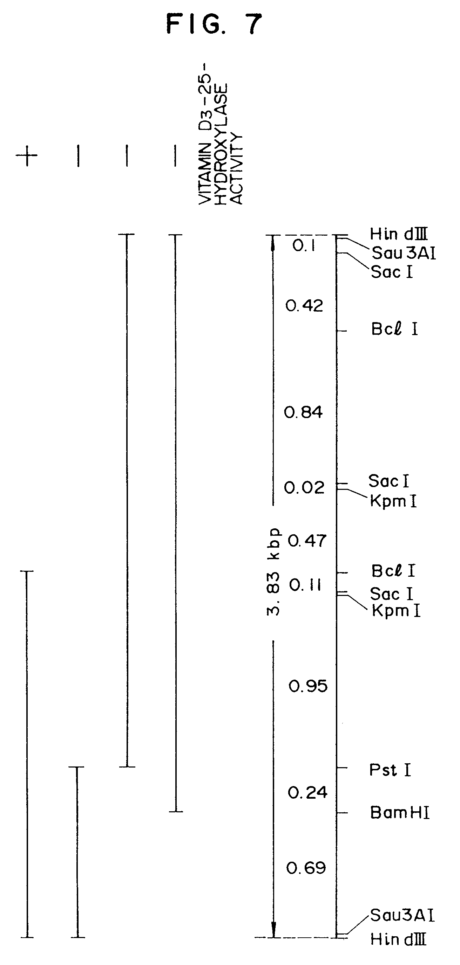

PHK030 is further subcloned and the active site is examined in a manner similar to the method shown in Example 2 later described. The examination reveals that vitamin D₃ 25-hydroxylase activity is located in the fragment of 2490 bp from the Sac I site at 1340 bp to the Hin dIII site at 3830 bp, from the upstream (cf. Fig. 7). From the results it is suggested that the vitamin D₃ 25-hydroxylation activity would be present in the open reading frame shown in Figs. 5 and 6.

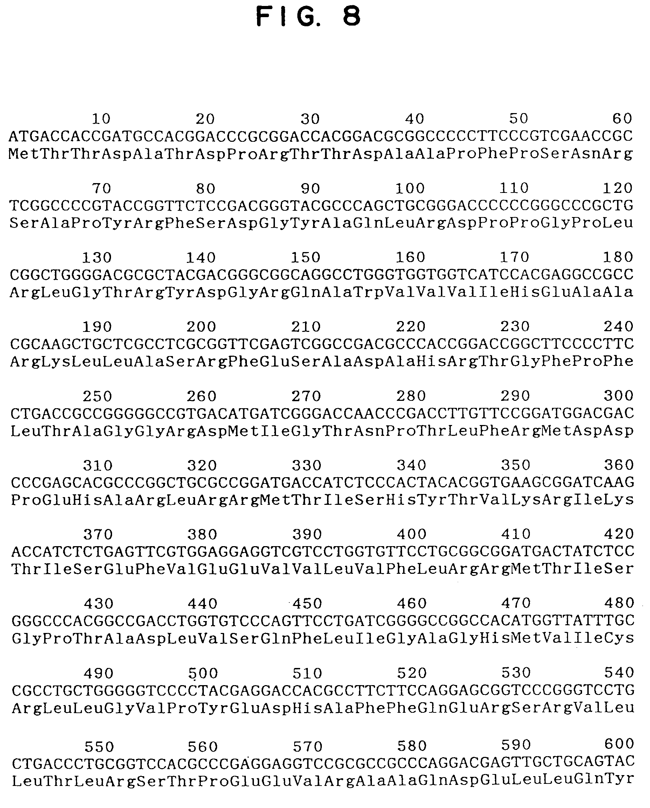

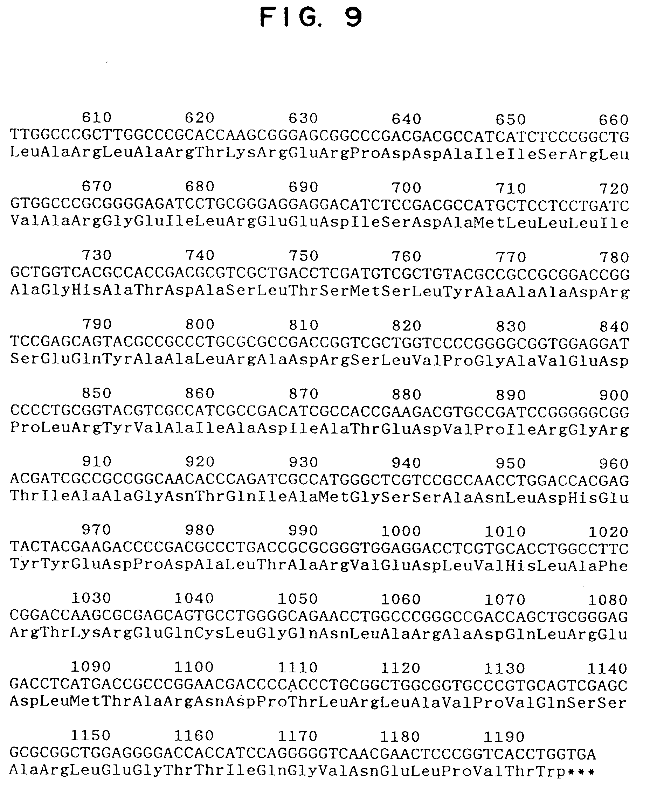

The foregoing reveals that the gene encoding the hydroxylase has the nucleotide sequence shown in Figs. 8 and 9.

### Actinomycetes

Actinomycetes which is employed or obtained in the present invention is described below in detail.

### (1) Nocardia autotrophica N-102

This strain has been deposited in Fermentation Research Institute of the Agency of Industrial Science & Technology Japan, under Accession No. FERM BP-1573. Its bacteriological properties are described in detail in Japanese Patent Application KOKAI No. 2-469. The strain is sometimes classified into Amycolata autotrophica by chemical classification (Lechevalier et al.; Int. J. Syst. Bacteriol., 36, 29 (1986)).

### (2) Streptomyces lividans TA-220

This strain has been deposited in Fermentation Research Institute of the Agency of Industrial Science & Technology Japan, under Accession No. FERM P-12962 on May 20, 1992 and then transferred on June 14, 1993 to International Deposit pursuant to the Budapest Treaty, where Deposit No. FERM BP-4337 has been allotted. The strain coincides with Streptomyces lividans TK24. The TK24 strain is described in "Genetic Manipulation of Streptomyces", A Laboratory Manual, The John Innes Foundation, 1985 and has been widely used as a host of Actinomycetes all over the world.

### (3) Streptomyces griseus TA-221

This strain has been deposited in Fermentation Research Institute of the Agency of Industrial Science & Technology Japan, under Accession No. FERM P-12963 on May 20, 1992 and then transferred on June 14, 1993 to International Deposit pursuant to the Budapest Treaty, where Deposit No. FERM BP-4336 has been allotted. The strain coincides with Streptomyces griseus HH-1 which is a mutant originated from Streptomyces griseus IFO 13350 (preserved by Institute for Fermentation, OSAKA). The bacteriological properties of the strain is clarified (HH-1 strain: Horinouchi et al., J. Bacteriol., 158, 481 (1984)). Streptomyces griseus IFO 13350 and the present strain are sometimes classified into Streptomyces bikiniensis.

### (4) Streptomyces lividans TA-222

This strain is a transformant bearing recombinant plasmid PHK030 obtained by the present invention, which corresponds to Streptomyces lividans TA-220 inserted with plasmid PHK030 therein.

### (5) Streptomyces griseus TA-223

This strain is a transformant bearing recombinant plasmid PHK031 obtained by the present invention, which corresponds to Streptomyces griseus TA-221 inserted with plasmid PHK031 therein.

### Production of 25-hydroxyvitamin D compounds using transformants

The 25-hydroxyvitamin D compounds are produced by reacting vitamin D compounds as substrate in a solution containing the transformant bearing the recombinant plasmid of the present invention or the hydroxylase produced by the transformants under aerobic conditions, using the transformants. The reaction may be carried out by a modification of the method described in, e.g., Japanese Patent Application KOKAI No. 2-469 using Actinomycetes. The 25-hydroxyvitamin D compounds can be produced irrespective of the presence or absence of soybean-derived substances, e.g., soybean powders or soy peptone, in a medium cultured to obtain the mycelia of the transformants necessary for the reaction. That is, even though any substance derived from soybean powders are not particularly supplemented to the medium, the hydroxylation activity of the transformants is expressed so that the 25-hydroxyvitamin D compounds can be prepared efficiently.

Hereinafter the present invention is described in more detail, with reference to the examples and experiments.

### Example 1 Production of recombinant plasmid and transformation

Nocardia autotrophica strain N-102 was inoculated on 100 ml of 0.5% glycine-containing tryptic soy liquid medium (Difco, USA) charged in a Sakaguchi flask of 500 ml, followed by incubation at 28°C for 4 days. The culture broth was centrifuged (500 rpm, 20 minutes) to collect the mycelia. The total DNA was extracted and purified from the cells by a modification of the Marmur method (J. Mol. Biol., 3, 208 (1961)), purified and dialyzed to TES buffer (50 mM Tris-hydrochloride, pH 8) for 16 hours to obtain donor DNA.

By reacting 5 µg of the thus obtained donor DNA at 37°C with restriction enzyme Sau 3AI (enzyme activity of 3 units, manufactured by Takara Shuzo Co., Ltd.; hereinafter restriction enzymes manufactured by Takara Shuzo Co., Ltd. or Wako Pure Chemical Industry Co., Ltd. were used and the reaction conditions were set forth according to the brochure attached, unless otherwise indicated in the examples), partial digestion was carried out to obtain DNA fragments of 3 to 20 kb (reaction time: 45 seconds). The reaction mixture was treated at 70°C for 15 minutes for inactivation. The reaction mixture was filtered through DE81 paper (Watmann) to recover DNA of 3 kb or larger.

Next plasmid PHK010 was prepared from PIJ699 (cf. Fig. 2). After reacting at 37°C for 2 hours in a buffer solution (H buffer; 50 mM Tris-hydrochloride, pH 7.5, 10 mM magnesium chloride, 1 mM dithiothreitol, 10 mM sodium chloride) containing 5 µg of PIJ699, 20 units of restriction enzyme Bgl II and 20 units of restriction enzyme Bam HI, the reaction mixture was subjected to ethanol precipitation (which is a treatment for recovering the DNA fragment by adding 2.5-fold amount of ethanol at -20°C, allowing the mixture to stand at -70°C for 30 minutes and then centrifuging at 15,000 rpm for 10 minutes) to recover the DNA fragment. After this DNA fragment was dissolved in 100 µl of TE buffer (10 mM Tris-hydrochloride, pH 8, 1 mM EDTA), the solution was subjected to BAP treatment (enzymatic reaction in a solution containing 100 µl of DNA solution, 12 µl of 1 M Tris-hydrochloride (pH 9), 4 µl (2 units) of bacterial alkaline phosphatase (E. coli C75, Takara Shuzo Co., Ltd.) and 4 µl of water) at 37°C for 2 hours. Following the reaction, the reaction solution was subjected to 0.8% agarose gel electrophoresis (apparatus for electrophoresis: Model TAKARA HE-13, agarose; Seakem Agarose ME (Takara Shuzo Co., Ltd.), buffer; 40 mM Tris-acetate, 20 mM sodium acetate, 2 mM EDTA, 18 mM NaCl, pH 8) and the DNA fragment (Fig. 2, (2)) corresponding to plasmid PHK010 (Fig. 3, (3)) of about 5 kb was recovered by DE81 paper. Plasmid PHK010 can be isolated by treating this DNA fragment with T4 ligase but the DNA fragment was employed for the following preparation of recombinant plasmid, without isolation.

The DNA fragment (1 µg) corresponding plasmid PHK010 was reacted at 4°C for 16 hours with the chromosomal DNA fragment (5 µg) obtained by treatment with restriction enzyme Sau 3AI, in 20 µl of LG buffer (30 mM Tris-hydrochloride, pH 7.5, 6 mM magnesium chloride, 10 mM dithiothreitol, 50 µg/ml bovine serum albumin, 0.5 mM ATP) containing 17.5 units of T4 DNA ligase. By this treatment recombinant plasmids were obtained in the reaction solution. The product is a mixture of a plasmid bearing the desired gene and capable of expressing the hydroxylase activity and a plasmid having no hydroxylase activity. In order to select the desired recombinant plasmid from the mixture of recombinant plasmids, the recombinant plasmid mixture was transfected to protoplast of Streptomyces lividans TA-220 which belongs to Actinomycetes inherently incapable or having an extremely poor ability of converting vitamin D compounds into 25-hydroxyvitamin D compounds.

That is, Streptomyces lividans TA-220 was shake cultured at 30°C for 48 hours in 100 ml of YEME medium containing 1% glucose, 34% sucrose, 0.3% yeast extract, 0.3% maltose extract, 0.5% peptone, 0.6% glycine and 5 mM magnesium chloride, which was charged in a Sakaguchi flask of 500 ml. The culture broth was centrifuged at 3,000 rpm for 10 minutes to collect the mycelia. The cells were suspended in and washed with 350 mM sucrose solution. The suspension was centrifuged and the collected mycelia were resuspended in 10 ml of L buffer ("LYSIS BUFFER"; described in "Genetic Manipulation of Streptomyces", A Laboratory Manual, The John Innes Foundation, 1985) and 200 mg of lysozyme was added to and dissolved in the suspension. The reaction at 30°C for an hour gave the protoplast of the strain TA-220. The reaction mixture was filtered through defatted cotton since the protoplast and the unreacted cells were present there as admixture. The filtrate was centrifuged at 3,000 rpm for 10 minutes to obtain precipitates abundant with the protoplast. The precipitates were suspended in 3 ml of L buffer. The suspension was added with 7.5 ml of glycerol to become the protoplast solution preservable at -20°C over long periods of time.

After 20 µl of the aforesaid solution of the recombinant plasmid mixture was added to 50 µl of this protoplast solution at 0°C, the mixture was mildly agitated to homogeneously disperse the protoplast. A solution containing 500 µl of 25% polyethylene glycol #1000, 2.5% sucrose, 0.025% potassium sulfate, 0.005% potassium dihydrogenphosphate, 10 mM calcium chloride and 50 mM Tris-maleate, pH 8, was added to the dispersion followed by mild agitation. After the agitation, the solution was mixed with 50 ml of soft agar medium (10.3 g of sucrose, 1 g of magnesium chloride hexahydrate, 0.294 g of 25 mM Tris-hydrochloride, pH 8, 0.65 g of agar). On regeneration medium charged in a petri dish, 3 ml each of the resulting mixture was overlaid. The regeneration medium was prepared by supplementing 6 ml of 0.5% potassium dihydrogenphosphate, 48 ml of 3.68% calcium chloride and 60 ml of 0.25 M Tris-hydrochloride buffer (pH 6) to 480 ml of a solution containing 61.8 g of sucrose, 6.0 g of glucose, 0.15 g of potassium sulfate, 6.07 g of magnesium hexahydrate, 0.06 g of Casamino acid, 3.0 g of yeast extract, 1.2 ml of a trace metal salt solution (40 mg/l zinc chloride, 200 mg/l ferric chloride hexahydrate, 10 mg/l cupric chloride dihydrate, 10 mg/l manganese chloride tetrahydrate, 10 mg/l sodium tetraborate decahydrate, 10 mg/l ammonium heptamolybdate tetrahydrate), 1.8 g of L-proline and 13.2 g of agar, autoclaving the mixture and then charging of 27 ml aliquots in the petri dish. After overlaying the soft agar medium containing the protoplast solution on the regeneration medium, incubation was carried out at 28°C for 16 hours. After the incubation, a solution (42°C) added with thiostrepton in the final concentration of 200 µg/ml was overlaid on the soft agar medium followed by incubation at 28°C for further 72 hours. By the above procedures, colonies containing 200 transformants per petri dish were obtained.

### Example 2 Screening of transformant capable of catalyzing hydroxylation of vitamin D compounds at the 25-position to convert into 25-hydroxyvitamin D compounds

The cells from the respective colonies of transformants obtained in Example 1 were inoculated, respectively, on 10 ml of a medium for microorganism conversion (1.5% glucose, 1.5% bacto-soyton (Difco), 0.5% corn steep liquor, 0.5% sodium chloride, pH 7.3) charged in a large test tube (25 mm φ), followed by aerated spinner culture at 28°C for 3 days. A solution of 1 mg of vitamin D₃ in 50 µl of ethanol was added to the culture broth, together with 50 µl of Tween 80. After the addition, aerated spinner culture was conducted again at 28°C for 3 days. After completion of the incubation, the culture broth was extracted by the Bligh & Dyer method (J. Lipid Res., 7, 739 (1966)). The extract of the methylene chloride layer was subjected to high performance liquid chromatography (HPLC, column; Zorbax-SIL, 4.6 mm φ x 25 cm, solvent; n-hexane : 2-propanol = 3 : 22, device: SHIMADZU LC-4A). As the result, transformants showing the peaks which retention time coincided with that of authentic 25-hydroxyvitamin D₃ (Duphar Co., Holland) were selected from the transformants tested, as a primary screening.

Next, the strains selected by the primary screening were inoculated on 50 ml of a medium for microbial conversion charged in a Sakaguchi flask of 500 ml, followed by fermentation with aeration and agitation at 28°C for 3 days. A solution of 5 mg of vitamin D₃ in 250 µl of ethanol was added to the culture broth, together with 250 µl of Tween 80. Fermentation with aeration and agitation was carried out at 28°C for further 3 days. After completion of the incubation, the fermentation broth was extracted by the Bligh & Dyer method. The extract of the methylene chloride layer was subjected to high performance liquid chromatography (HPLC, column; Zorbax-SIL, 4.6 mm φ x 25 cm, solvent; n-hexane : 2-propanol = 3 : 22, device: SHIMADZU LC-4A) using a regular phase column. The peak which residence time coincided with that of authentic 25-hydroxyvitamin D₃ (Duphar Co., Holland) was fractionated as the product. The product was subjected to high performance liquid chromatography (HPLC, column; Zorbax-CDS, 4.6 mm φ x 25 cm, solvent; water : methanol = 1 : 9, device: SHIMADZU LC-4A) using a reverse phase column. The peak which retention time coincided with that of authentic 25-hydroxyvitamin D₃ (Duphar Co., Holland) was fractionated and measured with respect to UV absorption spectrum (using spectrophotometer of Hitachi 220A) and mass spectrum (using a mass spectrometer of EI-MS, JEOL JMS-SN102). The results of the secondary screening above reveal that the retention time, UV absorption spectrum and mass spectrum pattern of the product from Streptomyces lividans strain TA-222 fully coincided with those of authentic 25-hydroxyvitamin D₃ (Duphar Co., Holland). It has thus been confirmed that the product was found to be 25-hydroxyvitamin D₃ (Method in Enzymology, 123, 127 (1986)), namely, Streptomyces lividans strain TA-222 was found to be the transformant capable of catalyzing the hydroxylation of vitamin D compounds to convert into 25-hydroxyvitamin D compounds.

### Product of Streptomyces lividans TA-222:

(1) Zorbax-SIL high performance liquid chromatography:
   Size of column: 4.6 mmφ x 25 cm
   Eluent: n-hexane : 2-propanol = 3 : 22
   Temperature of column: 25°C
   Velocity of elution: 1.5 ml/min
   Measured by SHIMADZU LC-4A
   Residence time of the authentic product: 4.0 minutes
(2) Zorbax-ODS high performance liquid chromatography:
   Size of column: 4.6 mmφ x 25 cm
   Eluent: water : methanol = 1 : 9
   Temperature of column: 40°C
   Velocity of elution: 1.0 ml/min
   Measured by SHIMADZU LC-4A
   Residence time of the authentic product: 8.0 minutes
(3) Maximum UV absorption:
   λₘₐₓ = 265 nm (in ethanol)
(4) EI-MS (m/z):
   400 (M⁺), 382 (M⁺ - H₂O), 271, 253, 136, 118, 59

### Example 3 Production of plasmid PHK030 by incubation of transformant Streptomyces lividans TA-222 and retransformation of Streptomyces lividans TA-222

By the procedures for screening described in Example 2, the transformant, Streptomyces lividans TA-220 was selected as the strain capable of catalyzing the hydroxylation of the 25 position of vitamin D compounds to convert into 25-hydroxyvitamin D compounds. This is considered to be because this strain contains a specific plasmid (plasmid PHK030) and is thus capable of converting vitamin D₃ into 25-hydroxyvitamin D₃. For confirmation, this plasmid was prepared and Streptomyces lividans was retransformed. That is, Streptomyces lividans TA-222 was inoculated on 50 ml of YEME medium shown in Example 1, which was charged in a Sakaguchi flask of 500 ml, followed by aerated spinner culture at 28°C for 72 hours. The culture broth was centrifuged (3,000 rpm, 20 minutes) to collect the mycelia. Crude plasmid mixture was extracted from the collected cells by the known neutral lysis method (Neutral Lysis: described in "Genetic Manipulation of Streptomyces", A Laboratory Manual, The John Innes Foundation, 1985), page 82) to obtain as the crude plasmid mixture solution in TES buffer. The solution was then purified by cesium chlorideethidium bromide equilibrium density gradient centrifugation (described in "Molecular Cloning", A Laboratory Manual, Second Edition, Cold Spring Harbor Laboratory Press, 1989, column 1, page 42). Plasmid PHK030 was thus isolated in the pure form as a closed circular plasmid.

The size of the inserted DNA fragment in recombinant plasmid PHK030 was determined by 0.8% agarose gel electrophoresis. That is, when 0.5 µg of recombinant plasmid PHK030 was digested with restriction enzyme Bam HI, recombinant plasmid PHK030 was digested at one site to give a straight chain DNA, whereby its size can be appreciated. The size of plasmid PHK030 was thus found to be about 8.5 kbp. When restriction enzyme Hin dIII was employed, plasmid PHK030 was digested at two sites to give DNA fragments of 3.8 kbp and 4.7 kbp, respectively. Using as a molecular weight marker the digestion fragments of λDNA with restriction enzyme Hin dIII or the digestion fragments of φX174 DNA with Hae III, the size of each DNA fragment was determined by digestion of plasmid PHK030 with Bam HI, based on the mobility of each electrophoresis. In a similar manner, plasmid PHK030 was digested with restriction enzymes Sac I, Bcl I, Kpn I and Pst I, respectively, and a restriction map was prepared based on the size of each fragment. The restriction map is shown in Fig. 3. Plasmid PHK010 (Fig. 2, (3)) has two sites for digestion with Hin dIII. This accordingly reveals that the plasmid has the site for digestion with restriction enzyme Sau 3AI within several tens bp from the both termini of the 3.8 kbp DNA fragment obtained by digestion of plasmid PHK030 with Hin dIII, and that the gene for hydroxylase derived from the chromosome of Nocardia autotrophica is contained in the region inward the digestion site. Another DNA fragment obtained by digestion with Hin dIII is a region derived from plasmid PHK010 (5.0 kbp, Fig. 2 (3)). It was found that this fragment is contained in PHK030 as the DNA fragment lost by 200 to 300 bp through recombination.

Using the thus obtained recombinant plasmid PHK030, Streptomyces lividans TA-220 was re-transformed by the procedures described in Example 1. With respect to 20 transformants obtained by the re-transformation, the ability of catalyzing vitamin D₃ to 25-hydroxyvitamin D₃ was examined. This ability was noted in all of the transformants. The plasmid isolated from the transformants was plasmid PHK030.

### Example 4 Removal of plasmid from transformant Streptomyces lividans TA-222 and re-transformation by plasmid PHK030

From the transformant, Streptomyces lividans TA-222, which came to acquire the ability of catalyzing the hydroxylation of the 25-position of vitamin D compounds to convert into 25-hydroxyvitamin D compounds by incorporation of plasmid PHK030 therein, plasmid PHK030 was deleted and removed by acriflavine or protoplast regeneration to obtain strain TA-222D. This strain was sensitive to thiostrepton because plasmid PHK030 was removed. Furthermore, the strain was examined in a manner similar to the procedures described in Example 2 to see if the strain is capable of converting into 25-hydroxyvitamin D₃. No conversion ability was noted with the strain.

Then, the plasmid-deleted strain TA-222D was transformed in a manner similar to the procedures described in Example 1, using plasmid PHK030. The re-transformant obtained by this transformation changed to thiostrepton resistance. The ability to convert into 25-hydroxyvitamin D₃ was also examined in a manner similar to the procedures described in Example 2. It was verified that the conversion ability was restored.

### Example 5 Preparation of plasmid PHK031

The DNA fragment obtained by digestion and linear-fashioning of plasmid PIJ702 with restriction enzyme Bgl II and the DNA fragment of about 3.8 kbp obtained by digestion of plasmid PHK030 with restriction enzyme Sau 3AI were ligated with each other using T4 DNA ligase in a manner similar to the procedures described in Example 3 to newly produce plasmid PHK031 having the size of 9.5 kbp. The size and restriction map of plasmid PHK031 shown in Fig. 4 were determined/prepared in a manner similar to Example 3.

### Example 6 Construction of transformant Streptomyces griseus TA-223 by plasmid PHK031 and the ability of converting vitamin D₃ to 25-hydroxyvitamin D₃

As a host microorganism to which the resulting plasmid PHK031 was to be transfected, there was used protoplast of Streptomyces griseus TA-221 which belongs to Actinomycetes inherently incapable or having an extremely poor ability of converting vitamin D compounds into 25-hydroxyvitamin D compounds. Streptomyces griseus TA-223 was obtained in a manner similar to the procedures described in Example 1, as the plasmid PHK031-transfected transformant. The ability of Streptomyces griseus TA-223 to convert vitamin D₃ into 25-hydroxyvitamin D₃ was examined in a manner similar to the procedures described in Example 2. The conversion ability of the transformant was recognized.

The DNA fragment of about 3.8 kbp obtained by digestion of plasmid PHK031 with restriction enzyme Sau 3AI and the DNA fragment obtained by digestion and linear-fashioning of plasmid PHK010 with restriction enzyme Bgl II were ligated with each other. The plasmid which has the same restriction map as that of plasmid PHK030 and fully coincides with plasmid PHK030 was obtained. Furthermore, the hydroxylation activity of vitamin D compounds was confirmed both in plasmid PHK030 and PHK031, indicating that the gene for hydroxylase was contained in the DNA fragment of about 3.8 kbp obtained by reacting plasmid PHK030 with restriction enzyme Hin dIII.

### Example 7 Conversion of vitamin D₂ into 25-hydroxyvitamin D₂ and conversion of 1α-vitamin D₃ into 1α,25-dihydroxyvitamin D₃, by transformant

1) Using the transformant Streptomyces lividans TA-222, 25-hydroxyvitamin D₂ was obtained from vitamin D₂ in a manner similar to Example 2. The thus obtained 25-hydroxyvitamin D₂ was perfectly identical with the authentic 25-hydroxyvitamin D₂ obtained by conversion of vitamin D₂ using Nocardia autotrophica in retention time of high performance liquid chromatography, UV absorption spectrum and mass spectrum pattern.
   (1) Zorbax-SIL high performance liquid chromatography:
      Size of column: 4.6 mmφ x 25 cm
      Eluent: n-hexane : 2-propanol = 3 : 22
      Temperature of column: 25°C
      Velocity of elution: 1.5 ml/min
      Measured by SHIMADZU LC-4A
      Retention time of the authentic product: 3.9 minutes
   (2) Maximum UV absorption:
      λₘₐₓ = 265 nm (in ethanol)
   (3) EI-MS (m/z):
      412 (M⁺), 394 (M⁺ - H₂O), 271, 253, 136, 118, 59
2) Using the transformant Streptomyces lividans TA-222, 1α,25-dihydroxyvitamin D₃ was obtained from 1α-vitamin D₃ in a manner similar to Example 2. 1α,25-Dihydroxyvitamin D₃ thus obtained was perfectly identical with the authentic 1α,25-dihydroxyvitamin D₃ in retention time of high performance liquid chromatography, UV absorption spectrum and mass spectrum pattern.
   (1) Zorbax-SIL high performance liquid chromatography:
      Size of column: 4.6 mmφ x 25 cm
      Eluent: n-hexane : 2-propanol = 3 : 22
      Temperature of column: 25°C
      Velocity of elution: 1.5 ml/min
      Measured by SHIMADZU LC-4A
      Retention time of the authentic product: 14.5 minutes
   (2) Maximum UV absorption:
      λₘₐₓ = 265 nm (in ethanol)
   (3) EI-MS (m/z):
      416 (M⁺), 394 (M⁺ - H₂O), 380 (M⁺ - 2H₂O), 287, 269, 251, 152, 134, 129, 116, 111, 59
3) Using the respective transformants and Nocardia autotrophica N-192, vitamin D₃ was converted into 25-hydroxyvitamin D₃. The yield of 25-hydroxyvitamin D₃ produced is shown in the following table. Time for conversion was 72 hours and the amount of 25-hydroxyvitamin D₃ produced was compared in each transformant.

**Table 1**

| Microorganism | Amount of 25-Hydroxyvitamin D₃ produced (µg/ml medium) |
|---|---|
| Nocardia autotrophica N-102 | 16.7 |
| Streptomyces lividans TA-222 | 20.4 |
| Streptomyces griseus TA-223 | 40.0 |

### Example 8 Determination of DNA fragment of 3.83 kbp cloned on PHK030

Using a variety of restriction enzymes, 1 µg of the DNA fragment of 3.8 kbp cloned on PHK030 was digested in a size of 800 bp or smaller. The thus obtained DNA digestion product, 0.5 µg of M13mp18RF (Takara Shuzo) digested with the same restriction enzyme and 0.5 µg of M13mp19RF (Takara Shuzo) were reacted at 4°C for 16 hours in 20 µl of LG buffer (30 mM Tris-hydrochloride, pH 7.5, 6 mM magnesium chloride, 10 mM dithiothreitol, 50 µg/ml bovine serum albumin, 0.5 mM ATP) containing T4 DNA ligase (8 units). By this reaction, recombinant DNA was obtained in the reaction mixture. The recombinant DNA was transfected to E. coli JM109 (C. Yanisch-Perrson, et al., Gene, 33, page 103 (1985)) treated with calcium chloride (Maniatis et al., Molecular Cloning, page 250 (1982)), which was then mixed with 2.5 ml of soft agar medium at 42°C containing 1 mM isopropyl-β-D-thiogalactopyranoside (IPTG, Takara Shuzo), 0.03% 5-bromo-4-chloro-3-indolyl-β-D-galactoside (X-gal, Takara Shuzo) and 0.2 ml of JM109 separately cultured (soft agar medium: 1% trypton, 0.5% yeast extract, 1% NaCl, 0.5% agar). The mixture was overlaid on 27 ml of L agar medium (1% trypton, 0.5% yeast extract, 1% NaCl, 1.5% agar) and incubated at 37°C for 16 hours to obtain plaque. The thus prepared plaque was adsorbed to JM109 and cultured. From the culture supernatant, single stranded DNA was extracted (Messing et al., Gene, 33, 103 (1985)) and the nucleotide sequence was determined according to the dideoxy chain terminator method (Sanger, F. et al., Proc. Natl. Acad. Sci. U.S.A., 74, 5463 (1977)), using SEQUENASE DNA Sequencing Kit (Toyobo Co.).

The results reveal that the DNA fragment cloned on PHK030 and derived from the chromosomal DNA of Nocardia autotrophica N-102 has a size of 3836 bp and contains 1194 bp open reading frame of 398 amino acids having initiation codon at 2300 bp from the upstream (Figs. 5 and 6).

Plasmid PHK030 was subcloned to examine the active site in a manner similar to the procedures described in Example 2. The results reveal that the 25 hydroxylase activity of vitamin D₃ is present in the 2490 bp fragment from the Sac I site at 1340 bp to the Hin dIII site at 3830 bp from the upstream (Fig. 7). This suggests that the 25-hydroxylation activity of vitamin D₃ would be present in the open reading frame shown in Figs. 5 and 6 (region translated into amino acids).

The foregoing results reveal that the gene encoding hydroxylase has the nucleotide sequence shown in Figs. 8 and 9.

### Industrial Applicability

Genes for converting the hydrogen atom at the 25-position of vitamin D compounds having hydrogen atom at the 25-position into a hydroxyl group to produce 25-hydroxyvitamin D compounds were hitherto found only in higher animal like a rat.

The present invention provides the gene for expressing a hydroxylase activity by converting the 25-hydrogen atom at 25-position of vitamin D compounds having hydrogen atom at the 25-position to produce 25-hydroxyvitamin D compounds, which gene is originated from a microorganism and has a different structure from those found so far in higher animal. In relation to the gene encoding a hydroxylase capable of converting the hydrogen atom at the 25-position of vitamin D compounds having hydrogen atom at the 25-position to produce 25-hydroxyvitamin D compounds, the present invention also provides the recombinant plasmid containing the DNA fragment of the gene. The present invention further provides transformants obtained by transfecting the recombinant plasmid containing the DNA fragment of the gene to another microorganism. The transformed microorganism can convert vitamin D compounds having hydrogen atom at the 25-position into 25-hydroxyvitamin D compounds, more efficiently than the strain originating the gene therefrom. Accordingly, the transformed microorganism is applicable to a process for manufacturing vitamin D compounds such as vitamin D₃ in the active form.

By introducing the recombinant plasmid bearing the gene provided by the present invention into its original strain of the gene or into a microorganism capable of catalyzing the hydroxylation of vitamin D compounds (e.g., microorganisms described in Japanese Patent Application KOKAI Nos. 2-469 and 2-231089), strains having an efficient conversion rate or strains having a shorter time for conversion per unit substrate (vitamin D compounds) can be obtained by an amplifying effect of the gene. Alternatively, it is expected to breed strains capable of efficiently producing 1α,25-dihydroxyvitamin D compounds, in harmony with the hydroxylation of vitamin D compounds at the 1α position possessed by the microorganism.

## Claims

1. A gene encoding a hydroxylase originated from Actinomycetes being capable of converting the hydrogen atom at the 25-position of a vitamin D compound having a hydrogen atom at the 25 position into a hydroxyl group, or a DNA bearing said gene.

2. A DNA according to claim 1, wherein said DNA bearing the gene encoding a hydroxylase is a DNA fragment of about 3.83 kb derived from chromosome of Actinomycetes which is capable of converting the hydrogen atom at the 25-position of a vitamin D compound having a hydrogen atom at the 25 position into a hydroxyl group, and which has a restriction map shown in Fig. 1.

3. A gene encoding a hydroxylase according to claim 1, wherein said gene encoding a hydroxylase codes for amino acid sequence shown in Figs. 8 and 9.

4. A gene encoding a hydroxylase or a DNA bearing the gene according to any one of claims 1 through 3, wherein said Actinomycetes is the genus Nocardia or the genus Amycolata.

5. A gene encoding a hydroxylase or a DNA bearing the gene according to any one of claims 1 through 4, wherein said Actinomycetes is the genus Nocardia autotrophica.

6. A recombinant DNA containing a gene encoding a hydroxylase or a DNA containing the gene according to any one of claims 1 through 5.

7. A recombinant DNA according to claim 6, which is capable of self-amplification in cells of a microorganism.

8. A recombinant DNA according to claim 7, which is plasmid PHK030 or PHK031.

9. A transformed microorganism bearing a recombinant DNA according to claim 7 or 8.

10. A transformed microorganism according to claim 9, wherein said transformed microorganism is Streptomyces lividans TA-222 or Streptomyces griseus TA-223.
